# EUROPEAN PATENT SPECIFICATION

(11) **EP 0 286 421 B1**
(45) Date of publication and mention of the grant of the patent: **18.05.1994**
(21) Application number: 88303150.2
(22) Date of filing: 08.04.1988
(51) Int. Cl.: A45D 2/36

(54) **Heat-generating material for portable hair curler**
Wärmeerzeugendes Material für Haarwickler
Matériau engendrant de la chaleur pour bigoudi protatif

(30) Priority: 10.04.1987 JP 53478/87; 16.07.1987 JP 108285/87
(43) Date of publication of application: 12.10.1988
(73) Proprietor: Nittetsu Fine Products Co., Ltd., Iwate (JP)
(72) Inventor: Ogawa, Yoshihiro, Kamaishi-shi Iwate (JP); Takahashi, Hiroo, Kamaishi-shi Iwate (JP)
(74) Representative: Arthur, Bryan Edward

(56) References cited:
- DE-C- 630 347
- DE-C- 637 457
- FR-A- 989 551
- US-A- 4 603 706

## Description

### (1) Field of the Invention

The present invention relates to a heat-generating material for a portable hair curler, and to a hair curler using this heat-generating material.

More particularly, the present invention relates to a heat-generating material for a portable hair curler, which does not damage the hair during curling thereof and can be supplied at a low cost, is easily handled, rapidly curls the hair, and can be thrown away after use, and to a hair curler using this heat-generating material.

### (2) Description of the Related Art

As is well-known, hair curlers of the type utilizing the generation of heat by electricity are conventionally used, but, for structural reasons, hair curlers of this type are heavy and bulky, and are not easily portable.

With the recent development of pocket heaters utilizing a metallic heat-generating agent, various portable hair curlers (hot curlers) utilizing such a metallic heat-generating agent have been proposed. However, these hair curlers have the following disadvantages:
(1) For example, in a hair curler of the type where the hair is directly wound onto a metallic heat-generating material comprising a metallic heat-generating agent contained in an air-permeable bag (see, for example, Japanese Unexamined Utility Model Publication No. 59-16501), an excessive heat-generating effect is given to the hair, and the hair is often damaged. Moreover, since the hair must be touched by the hands when curling, there is a fear that problems will arise due to a breakage of the metallic heat-generating material. Especially, since women users generally allow their nails to grow very long for reasons of fashion, the risk of a breakage of the metallic heat-generating material is very high.
(2) In a hair curler comprising a metallic heat-generating agent packed in a hollow cylinder onto which the hair is wound (see, for example, Japanese Unexamined Utility Model Publication No. 51-76782), since an element for fixing the hair is not included, generally the hair is not properly curled. Moreover, since the metallic heat-generating agent is packed in the cylinder, when the curler is to be used, it is difficult to promote the necessary oxidation for obtaining a heat-generating action by lightly shaking the metallic heat-generating material. Furthermore, since an element for fixing the hairs is not included, the operation of winding the hair is cumbersome, and the curling operation cannot be performed rapidly or easily.
(3) A hair curler in which the above-mentioned disadvantages are eliminated was proposed, for example, in Japanese Unexamined Utility Model Publication No. 51-81183. This hair curler is provided with a hair-fixing element, and thus the cumbersome operation of winding the hair is eliminated. However, since the composition of the metallic heat-generating agent is not specifically disclosed, it is difficult to ascertain whether it is possible to impart a moisturizing effect to the hair.

The reason why the composition of the heat-generating agent for generating heat by oxidation, as utilized for a hair curler, is not specified, is considered to be that this technique still needs much improvement and an effective composition has not been formulated as yet. Moreover, this non-clarification of the details of the composition leads one to assume that it is impossible to curl the hair while applying steam to the hair to attain a moisturizing effect, when this composition is used.

As other related arts, there can be mentioned Japanese Unexamined Utility Model Publication No. 54-151585 entitled "Throwaway Self-Heat-Generating Hair Curler", Japanese Unexamined Utility Model Publication No. 56-145402 entitled "Throwaway Hair-Winding Curler", Japanese Unexamined Utility Model Publication No. 60-94101 entitled "Hair Curler", and Japanese Examined Patent Publication No. 53-47744 entitled "Steam Hair Iron".

As can be seen from these prior arts, hair curler technology is rapidly developing.

The hair curler proposed in Japanese Examined Patent Publication No. 53-47744 is one of the most popular in use at present, but for structural reasons, the hair curler is not easily portable or easily used. The hair curler proposed in Japanese Unexamined Utility Model Publication No. 60-94101 is disadvantageous in that the hair curler is not easily used and the cost is high, because butane gas or the like is used.

The self-heat-generating curlers disclosed in Japanese Unexamined Utility Model Publication No. 54-151585, Japanese Unexamined Utility Model Publication No. 59-16501, and Japanese Unexamined Utility Model Publication No. 56-145402 are improved versions of the above-mentioned hair curlers, in that they handle easily and cost less, and users are able to carry and use them as portable hair curlers. In these self-heat-generating hair curlers, since water is removed from the hair wound on a hair-curling member, on the contact side, to attain a curling effect (the hair is bent or coiled), an unbalance in the water content of the hair often occurs. Accordingly, these hair curlers are defective in that damage to the hair is repeated and worsened when the hair is curled many times successively and thus it is difficult to maintain the hair in a healthy state.

Accordingly, these conventional portable hair curlers (hot curlers) have not been marketed because of these defects. Further, even if marketed, safe transportation or use cannot be guaranteed, and a hair curler which satisfies all of the requirements of a rapid operation, ease of handling, and lasting curling and moisturizing effects, has not been developed.

FR-A-989551, which forms the basis for the first part of claim 1, discloses a heat-generating agent comprising a hydrate and water, the two substances being mixed upon the breaking of a cartridge within a metal outer chamber.

DE-C 630347 discloses a heat generating agent which generates heat by oxidation.

DE-C 637457 discloses a heat generating agent comprising a mixture of reduction iron, and alkaline charate. None of the prior art referred to above discloses a heat-generating device in accordance with the present invention as hereinafter described.

### SUMMARY OF THE INVENTION

Therefore, a primary object of the present invention is to overcome the defects of the conventional techniques and provide a metallic steam-heat generating material for a portable hair curler, which satisfies all of the above-mentioned requirements.

Another object of the present invention is to provide a portable hair curler comprising this heat-generating material, in which the defects of the conventional portable hair curlers are eliminated.

Namely, according to the present invention, as claimed in claim 1, there is provided a heat-generating device for a portable hair curler which comprises a heat-generating agent comprising iron powder, water, a water-retaining agent for retaining the water and a metal halide, the heat-generating agent being contained in an air-permeable bag and the device being capable of generating a temperature of 70°C to 120°C and being capable of maintaining a temperature higher than 60°C for at least 20 minutes.

Further embodiments according to the invention are claimed in claims 2-13.
(1) Safety is ensured during transportation or during use. For example, the generation of unnecessary heat and damage such as a breaking of the hair curler are prevented.
(2) Easily portable
(3) The heat-generating material or hair curler is easy to handle.
(4) The price is very low (i.e., the price is such that the curler can be easily purchased).
(5) The metallic heat-generating material is disposable, i.e., throwaway type.
(6) Damage to the hair is minimized.
(7) A moisturizing effect is continuously given to the hair.
(8) The curling of the hair is completed within about 20 minutes.
(9) The metallic heat-generating materials are exchangeable.

### BRIEF DESCRIPTION OF THE DRAWINGS

Figure 1 is a perspective view of a metallic heat-generating material for a portable hair curler according to the present invention;
Fig. 2 is a perspective view of the entire structure of a portable hair curler comprising the metallic heat-generating material shown in Fig. 1;
Fig. 3 is a front view of an example of a commercial product of the portable hair curler according to the present invention;
Fig. 4 shows the temperature-elevation curves of the metallic heat-generating material according to the present invention and of a comparative metallic heat-generating material;
Fig. 5 shows the amount of water dissipated by the metallic heat-generating material according to the present invention and by the comparative metallic heat-generating material shown in Fig. 4;
Fig. 6 is a perspective view of another example of a commercial product of the portable hair curler according to the present invention;
Fig. 7 is a detailed perspective view of a hair-winding guide cylinder of the hair curler shown in Fig. 6;
Fig. 8(a) is a perspective view of the outer surface of a cover of the hair curler shown in Fig. 6; and,
Fig. 8(b) is a perspective view of the inner surface of a cover of the hair curler shown in Fig. 6.

### DESCRIPTION OF THE PREFERRED EMBODIMENTS

Note: Throughout the drawings, the same referential numerals represent the same members, as follows:
1, metallic heat-generating material; 2, air-permeable bag; 3, metallic heat-generating agent; 4, winding member; 5, hollow portion; 6, projection; 7, fine hole; 8, pressing member; 9, flange; 10, packaging bag (air-permeable); 11, portable hair curler; 12, hair-winding guide cylinder; 13, cap; 14, cover; 15, vent hole; 16, comb tooth-like projection; 17, clearance; 18, opening; 19, vent hole in cover; 20, projection; 21, air-tight bag or container.

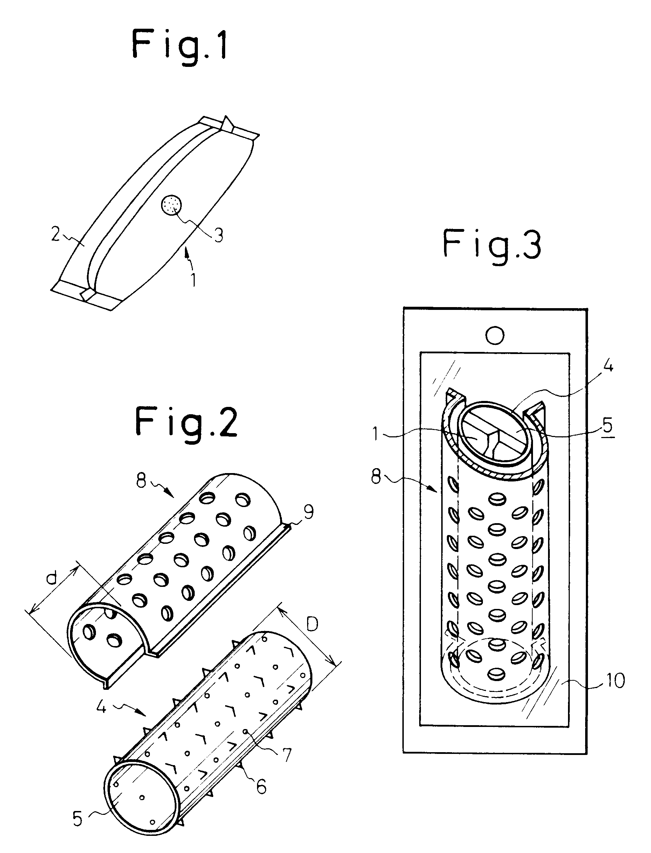

A metallic heat-generating material 1 for a portable hair curler according to one aspect of the present invention is shown in Fig. 1, wherein a part of the bag is cut away to illustrate the metallic heat-generating agent 3. Namely, this heat-generating material 1 comprises a metallic heat-generating agent 3, described in detail hereinafter, and an air-permeable bag 2 containing the metallic heat-generating agent 3 therein.

The bag 2 is composed of, for example, a nylon nonwoven fabric/polyethylene laminate having vent holes or a polyethylene nonwoven fabric (marketed under the tradename of "TYVEK"), having a good air-permeability and contributing to the temperature-elevating action and steam dissipating action of the metallic heat-generating material.

The metallic heat-generating agent 3 used in the present invention preferably has the following composition:
Iron powder: 40 to 75%
Zeolite: 15 to 25%
NaCl: 2 to 5%
NaOH: 0.1 to 0.5%
Active carbon: 1 to 3%
Water: 14 to 20%
In this metallic heat-generating agent, the water is converted to steam by an oxidation or hydration reaction, and steam is dissipated from the bag, and therefore, a continuous moisturizing effect is obtained.

The reasons for the above limitation of the composition of the metallic heat-generating agent will now be described.

The reason for the limitation of the iron powder content to 40 to 75% is first described.

In the present invention, iron powder is a substance to be oxidized, and the mixing ratio and amount of iron powder should be such that the quantity of heat necessary for curling is obtained, that is, a highest temperature of 70 to 120°C and a duration of heat at a temperature higher than 60°C of at least 20 minutes. Furthermore, a good balance must be maintained between the amount of iron powder and the amount of water added as an oxidant, and the amount of water-retaining agent for retaining water and various salts added thereto. To ensure an easy portability and handling ease, the amounts of the constituents of the heat-generating agent must be within the above-mentioned ranges.

Accordingly, when the iron powder content is at a lowest level of 40%, the iron powder mixing ratio is low, and excessive amounts of the oxidizing agent and the like are incorporated. Preferably, the amount of iron powder is such that water is contained in a theoretically necessary amount, the water-retaining agent is contained in an amount corresponding to the necessary amount of water, and a salt as an oxidation speed-adjusting agent is contained in a necessary minimum amount, and that this value is changed in accordance with the kind of iron powder used. If the iron powder mixing ratio is lower than 40%, the oxidant or the like is wastefully lost, the apparent density is unnecessarily reduced, and the bulk is increased, with the result that the intended objects of the present invention cannot be realized.

Preferably, the iron powder mixing ratio is up to 75%. Note, an excess amount of iron powder does not contribute to the reaction and apparent heat is wastefully used for heating this excess of iron powder. However, where it is desired that curling be carried out at a relatively low temperature over a long time period, an increase of the iron powder mixing ratio effectively increases the entire heat capacity of the metallic heat-generating material. However, where the metallic heat-generating material is contained in a small volume, such as in a hair curler, the upper limit of the mixing ratio for the hair curler is 75%.

The reason for the limitation of the content of zeolite (e.g., marketed under the tradename "ZEOPHYL") to 15 to 25% is now described. The zeolite acts as a water-retaining agent for retaining the water necessary for oxidation (hydration) of the iron powder and 90% of the incorporated water is retained in the zeolite. Accordingly, the zeolite mixing ratio is determined according to the theoretically necessary amount of water.

The lower limit of the zeolite mixing ratio is restricted to 15% because if the mixing ratio of zeolite is lower than this lower limit, the amount of water retained by the unit amount of zeolite is increased, the starting material becomes wet, and the proportion of water dissipated in the form of steam is increased. The upper limit of the mixing ratio is restricted to 25% because, if the zeolite mixing ratio exceeds this upper limit, the amount of water retained by the unit weight of zeolite is reduced, the apparent density is reduced (to no purpose), and the heat-generating agent becomes bulky, which degrades the bag-preparing property.

The reason for the limitation of the halide (e.g., sodium chloride (NaCl)) mixing ratio to 2 to 5% is now described. In the metallic heat-generating material of the present invention, NaCl acts as a catalyst for promoting the oxidation of the iron powder. The present inventors studied the heat-generating characteristics necessary for a metallic heat-generating material for a hair curler and found that the elevation time required for the temperature to rise to 60°C is preferably 3 to 4 minutes and the maximum temperature is preferably 70 to 120°C. If the NaCl mixing ratio is lower than 2%, the elevation time is too long, i.e., 8 to 10 minutes, and if the NaCl mixing ratio exceeds 5%, the starting material becomes very wet and viscous. Therefore, the NaCl mixing ratio is limited to 2 to 5%.

The reason for the limitation of the agent for controlling the generation of hydrogen (e.g., sodium hydroxide (NaOH)) mixing ratio to 0.1 to 0.5% is now described. The NaOH controls the generation of hydrogen gas when the iron powder is oxidized (hydrated) by the metal halide.

If the NaOH mixing ratio is lower than 0.1%, hydrogen gas is generated, and when a pure iron powder is used, this generation of hydrogen gas is conspicuous. Even if NaOH is added in an amount exceeding 0.5%, the NaOH effect is not particularly increased, the elevation time as a heat-generating characteristic is prolonged, and the alkalinity of the heat-generating agent is increased. Preferably, the mixing ratio of NaOH is 0.3 to 0.4%.

The mixing ratio of active carbon is limited to 1 to 3% because, in the metallic heat-generating agent of the present invention, active carbon adsorbs the water-retaining agent and oxygen in the atmosphere. Namely, when heat is generated by the heat-generating material, the active carbon supplies the water necessary for oxidation, and oxygen from the atmosphere is introduced into the bag containing the metallic heat-generating agent by the active carbon to ensure a smooth heat-generating reaction.

If the active carbon mixing ratio is lower than 1%, a sufficient amount of oxygen is not supplied to the heat-generating agent and heat generation of the oxygen passage-determined rate in the package occurs. Accordingly, the lower limit of the active carbon mixing ratio is restricted to 1%. If the active carbon mixing ratio exceeds 3%, heat generation of the iron powder at the oxidation-determined rate occurs and the intended effect can not be realized. Accordingly, the upper limit of the mixing ratio of active carbon is restricted to 3%.

The reason for the limitation of the water mixing ratio to 14 to 20% is now described. In the iron powder type metallic heat-generating material, water acts as an oxidant of the iron, as summarized by the following reaction formula:

Fe + 3/4O₂ + 3/2H₂O = Fe(OH)₃

The amount of water necessary for the metallic heat-generating material can be theoretically calculated from the above reaction formula. However, if it is intended to obtain a high temperature of 70 to 120°C in a relatively short time, and to maintain a temperature higher than 60°C for about 20 minutes, as in the case of the hair curler of the present invention, the iron powder mixing ratio should be increased.

If only the temperature characteristics required for the heat-generating material are taken into consideration, about 50% at most of the theoretical amount of water necessary for oxidation of the incorporated iron powder is sufficient, which amount corresponds to 15% of the total amount of the metallic heat-generating agent.

Note, even if the amount of water is actually smaller by about 35% than the said 15% of the total amount of the metallic heat-generating agent, i.e., about 10% of the above agent, steam is generated.

This is because 40 to 70% of the incorporated iron powder is subjected to oxidation within a limited time.

Therefore, the theoretically necessary water amount is actually decided by the relationship to an oxidized iron powder amount, and steam is continuously generated by an amount of water amounting to more than the aforementioned approximately 10%.

In the present invention, a continuous generation of steam is desired, and therefore, the lower limit of water mixing ratio is restricted to 14%, which exceeds the theoretical amount of water necessary for actual oxidation of the iron powder.

If water is incorporated in an amount exceeding 20%, the starting material becomes wet and viscous and the time from the generation of heat in the metallic material to the time when the effect of this heat is felt is extended, and therefore, and the intended heat-generating characteristics cannot be obtained. Accordingly, the upper limit of the water mixing ratio is restricted to 20%. Preferably, the water mixing ratio is 14 to 17%.

Water is advantageously included in the water-retaining agent and active carbon. The water-inclusion method may be selected from among the following embodiments.

Namely, when water is incorporated into the air-permeable bag, the following embodiments can be adopted.
(a) Water is incorporated in the zeolite.
(b) A larger amount of water is incorporated in the active carbon.
(c) Water is separated from the metallic heat-generating agent in the bag and is mixed with the metallic heat-generating agent at the time of application.
(d) Sawdust is incorporated and water is included in this sawdust.
(e) A highly water-absorbent polymer is incorporated, and water is included in this polymer.

Note, an appropriate embodiment may be selected from among these embodiments.

As shown in Fig. 1, when the metallic heat-generating material 1 having the above-mentioned structure is used, the bag 2 is lightly shaken to mix the metallic heat-generating agent 3 and produce a reaction therein, and the bag 2 is then inserted in the hollow portion of the winding member 4.

Subsequent to the above process, as shown in Fig. 2, the hair is wound on the winding member 4 and the pressing member 8 is fitted thereover. Since the hair is gripped between the winding member 4 and the pressing member 8, the hair will remain in the set position until these members 4 and 8 are separated. Namely, since the outer diameter D of the winding member 4 is nearly equal to the inner diameter d of the pressing member 8 (D = d), movement of the hair is prevented.

If projections 6 are formed on the winding member 4, an appropriate clearance is formed in the wound hair during the winding of the hair, and an effective curling of the hair obtained.

As shown in Fig. 2, many fine holes are formed in the winding member 4 to allow a smooth dissipation of the steam, and thus obtain a continuous moisturizing effect. Namely, steam dissipated from the metallic heat-generating material 1 passes through the fine holes 7 and is impinged on the hair, whereby the steam, together with the elevated temperature (70 to 120°C), causes the hair to curl while maintaining a good water balance in the hair. The time required for this curling operation is about 20 minutes.

When the winding member 4 is to be removed from the hair, the flange 9 is expanded and the pressing member 8 is lifted off of the winding member 4. After completion of the curling operation, the metallic heat-generating material 1 is removed from the hollow portion 5 and thrown away, the pressing member 8 is fitted to the winding member 4 and these members are stored in this state until required for use again. If the members 4 and 8 are composed of a consumable material, they can be thrown away together with the metallic heat-generating material.

Figure 3 shows an example of a metallic heat-generating material 1 which is now commercially available. A packaging bag 10 is composed of a gas-barrier packaging material (for example, a vinylidene chloride-coated polypropylene or nylon/polyethylene laminate) or an aluminum foil/polyethylene laminate (aluminum foil packaging material). Accordingly, the metallic heat-generating material 1 is safely stored so that a reaction can not occur before use. Since the metallic heat-generating material 1 is inserted into the hollow portion 5 of the winding member 4 and the pressing member 8 is fitted thereto, during transportation the metallic heat-generating material 1 is easily handled and a high safety is guaranteed. Score lines are formed in the packaging bag 10 so that one heat-generating material or a group of heat-generating materials can be separately handled, and accordingly, an appropriate number of metallic heat-generating materials may be separated according to the intended use.

Moreover, the packaging bag 10 can be formed as one bag, and accordingly, an appropriate form can be chosen for the packaging bag 10 to be marketed, so long as the metallic heat-generating material 1 is contained in the bag 10 in the state wherein the metallic heat-generating material 1 is inserted into the hollow portion 5 of the winding member and the pressing member 8 is fitted to the winding member 4, and the intrusion of oxygen is prevented.

Another example of the metallic heat-generating material 1 will be now described. In this example, one metallic heat-generating material 1 is contained in a gas-barrier bag.

When this heat-generating materials is used, the gas-barrier bag is opened just before use, and the metallic heat-generating material 1 is taken out and filled into a hair-winding guide cylinder of a hair curler to be used. Namely, a metallic heat-generating material for filling is provided. Several metallic heat-generating materials for filling can be connectedly packaged in such a manner that they can be easily separated from one another.

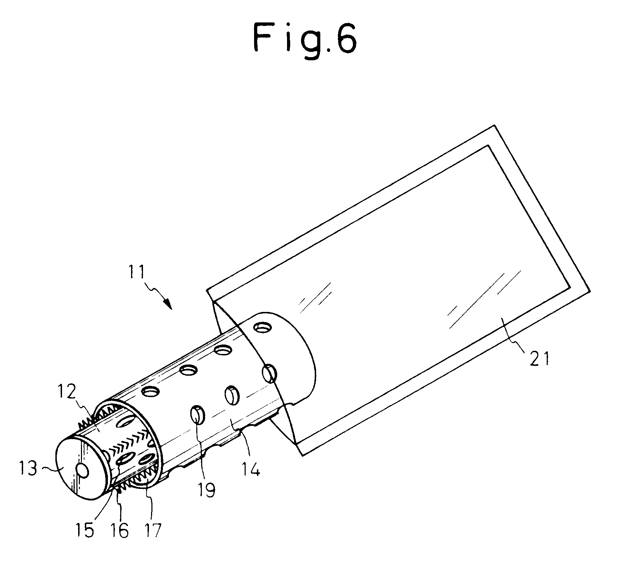

Figure 6 shows another embodiment of the portable hair curler according to the present invention.

Referring to Fig. 6, a portable hot curler 11 comprises a hair-winding guide cylinder 12, a cap 13, and a cover 14. The metallic heat-generating material 1 shown in Fig. 1 is inserted into the hair-winding guide cylinder 12. Members other than the metallic heat-generating agent 3 of the metallic heat-generating material 1 are composed of a synthetic resin.

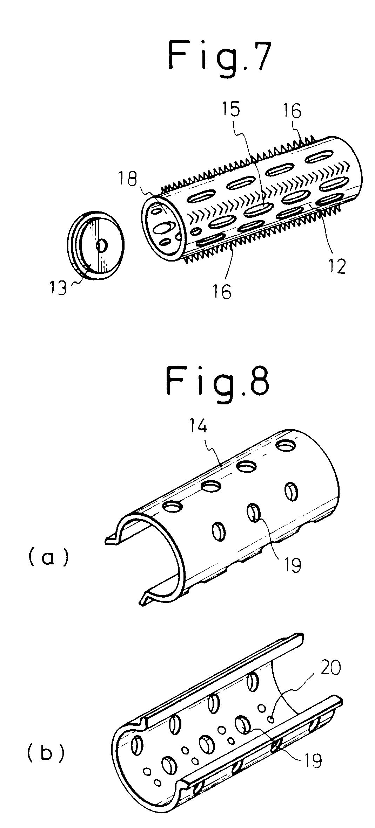

As shown in Fig. 3, the hair-winding guide cylinder 12 has a hollow cylindrical shape with vent holes 15 formed therein. Projections 16 resembling comb teeth, which extend in the longitudinal direction of the hair-winding guide cylinder 12, are formed at intervals on the outer surface of the cylinder 12 in the embodiment shown in Fig. 7. Six rows of projections 16 are arranged in this embodiment. When the hair-winding guide cylinder 12 is fitted to the cover 14, as explained hereinafter, the projections 16 resembling comb teeth prevent rotation of the hair-winding guide cylinder 12 and thus stabilize the winding of the hair. A clearance 17 is formed between the hair and the guide cylinder 12 so that the heat-generating action of the metallic heat-generating material 1 is appropriately exerted on the hair, and a continuous moisturizing effect by steam from the metallic heat-generating material 1 is easily obtained.

In the hair-winding guide cylinder 12, one end is closed in advance in the preparation process and an opening 18 is formed in the other end so that the metallic heat-generating member 1 can be exchangeably inserted therein. A cap 13 is fitted to that end to close the opening 18. Accordingly, the metallic heat-generating material 1 inserted in the hair-winding guide cylinder 12 can not fall out of the hair-winding guide cylinder 12 unless the cap 13 is removed. Accordingly, an unnecessary generation of heat can be prevented during transportation or use, and a high safety can be maintained. Note, a modification may be adopted in which both ends of the hair-winding guide cylinder 12 are open and caps are fitted to both openings to close the hair-winding guide cylinder 12.

As shown in Figs. 8(a) and 8(b), the cover 14 has vent holes 19 and four pairs of confronting projections 20 are arranged in the longitudinal direction on the inner wall of the cover 14. The projections 20 press against the above-mentioned projections 16 to prevent rotation of the hair-winding guide cylinder 12. The sectional shape of the cover 14 resembles the electric ohm symbol Ω, and elasticity is imparted by this Ω-like sectional shape. Accordingly, the hair-winding guide cylinder 12 can be turned by hand while fitted to the cover 14, and thus a good fitting state is maintained between the cover 14 and the hair-winding guide cylinder 12.

Furthermore, if the cover 14 is fitted to the hair-winding guide cylinder 12 as shown in Fig. 6, even when the cylinder 12 moves in the longitudinal direction of the cover 14, the hair-winding guide cylinder 12 will not separate from the cover 14 as it is held by the mutual action of the Ω-like sectional shape and the projections 16 formed on the hair-winding guide cylinder 12, unless released by hand, and therefore, a high safety can be maintained.

Before application, the metallic heat-generating material of the present invention is contained in a container vessel 21 such as an air-tight bag to prevent an unnecessary generation of heat, as shown in Fig. 6, and the metallic heat-generating material is marketed in this state.

At the time of application, the portable hair curler according to the present invention is removed from the bag 21, and the metallic heat-generating material 1 is lightly shaken to mix same. Then the hair is wound onto the hair-winding guide cylinder 12 containing the metallic heat-generating material 1, with air passing through from the vent holes 15 and 19, and the cover 14 is then fitted to the hair-winding guide cylinder 12. The temperature rapidly rises to about 70 to about 120°C, and the hair is curled. In this case, where water is contained in the composition of the metallic heat-generating material, this water is evaporated to impart a continuous moisturizing effect to the hair, and the hair is thus curled as desired.

The present invention will now be described in detail with reference to the following examples, that by no means limit the scope of the invention.

### Example 1

This example illustrates an embodiment of the metallic heat-generating material 1 in which a continuous moisturizing effect can be obtained and the hair is curled for a relatively long time. Since cast iron powder is used as the iron powder, the following excellent characteristics are obtained.
(1) The hair is slowly (about 20 minutes) curled at a relatively low temperature, (70 to 80°C).
(2) There is no feeling of heat.
(3) The curling temperature is low, and thus damage to the hair is minimized.

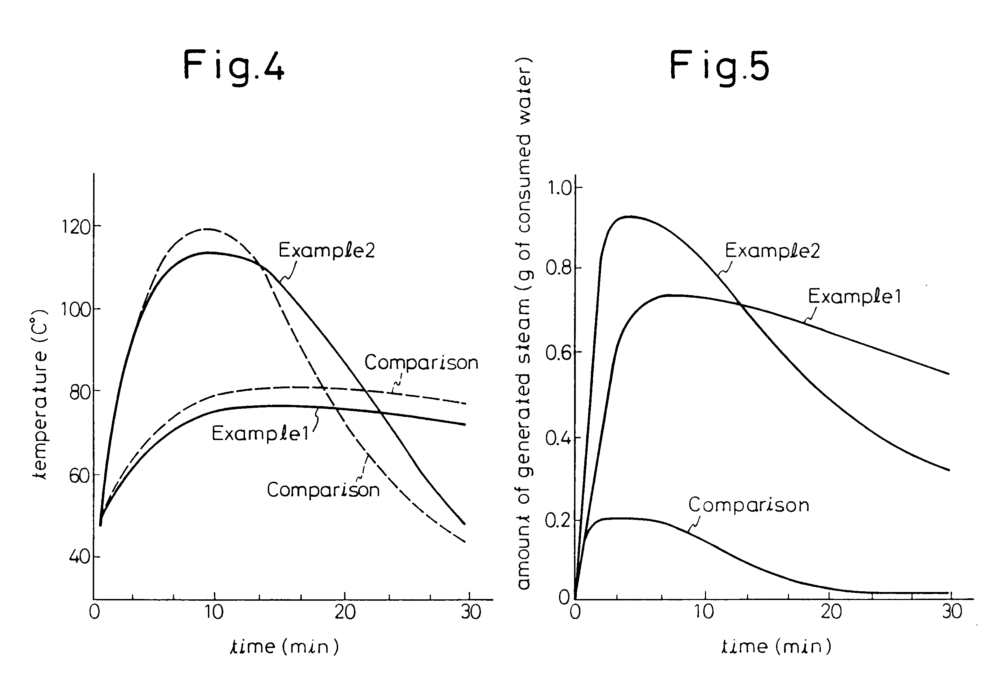

Figures 4 and 5 show the heat-generating characteristics and amounts of steam generated in the metallic heat-generating material of this example of the present invention and the comparative metallic heat-generating material not containing water. As is apparent from the results shown in Figs. 4 and 5, moist curls can be obtained in about 20 minutes in the case of the metallic heat-generating material of the present invention.

### [composition of metallic heat-generating agent]

Cast iron powder: 53.7% (the content of particles having a size smaller than 0.15 mm was 50%)
Zeolite: 21%
NaCl: 3%
NaOH: 0.3%
Active carbon: 2%
Water: 20%

### [method for inclusion of water]

Water was included in the zeolite, as shown in Fig. 1, and premixed with other components.

### [air-permeable bag 2]

Material: nylon nonwoven fabric/polyethylene laminate having through holes
Size: material thickness = 90 µ, height of bag = 5 mm , width of bag = 20 mm, length = 75 mm

### [winding member 4 and pressing member 8]

Commercially available products were used.

### Comparative Example 1

The comparison test was carried out in the same manner as described in Example 1 except that the composition of the metallic heat-generating agent was changed as described below.

Cast iron powder: 63.7% (the content of particles having a size smaller than 0.15 mm was 50%)
Zeolite: 21%
NaCl: 3%
NaOH: 0.3%
Active carbon: 2%
Water: 10%
As apparent from this composition, the water content was low and about 1/2 of the water content in Example 1 of the present invention. Accordingly, the dissipation of steam was reduced and a continuous moisturizing effect was not obtained.

### Example 2

This example illustration an embodiment of the metallic heat-generating material 1 in which a continuous moisturizing effect is attained and the hair can be curled in a short time. Since pure iron powder (pickled reduced iron powder) is used as the iron powder, the following excellent characteristics are obtained.
(1) The hair can be curled at a relatively high temperature of 100 to 120°C in a short time of 8 to 15 minutes.
(2) Although the curling temperature is relatively high, a good moisturizing effect is attained and the hair is not damaged.

Figures 4 and 5 show the heat-generating characteristics and amounts of steam generated in the metallic heat-generating material of this example according to the present invention and the comparative metallic heat-generating material not containing water. As seen from the results shown in Figs. 4 and 5, moist curls are obtained within about 15 minutes according to the present invention.

### [composition of metallic heat-generating material]

Iron powder (pickled reduced iron): 59.5% (the content of particles having a size smaller than 75 µ was 65%)
Zeolite: 21%
NaCl: 3%
NaOH: 0.5%
Active carbon: 2%
Water: 14%

### [method for inclusion of water]

Water was included in the zeolite and mixed with other components of the metallic heat-generating agent, and the mixture then filled in the air-permeable bag 2. The bag 2 was then promptly sealed in a gas-barrier bag to prevent contact with the outer air.

### [Air-Permeable Bag 2]

Material: Japanese paper/polyethylene laminate having holes formed only in the polyethylene
Size: material thickness = 75 µ, height of bag = 5 mm, width of bag = 20 mm, length of bag = 75 mm

### [winding member 4 and pressing member 8]

Commercially available products were used.

### Comparative Example 2

The comparison test was carried out in the same manner as described in Example 2 except that the composition of the metallic heat-generating material was changed as follows.
Iron powder (pickled reduced iron powder): 63.5% (the content of particles having a size smaller than 75 µ was 65%).
Zeolite: 21%
NaCl: 3%
NaOH: 0.5%
Active carbon: 2%
Water: 10%
As apparent from the above composition, the water content was low and about 1/2 of the water content in Example 2 according to the present invention, and therefore, as shown in Fig. 5, dissipation of the steam was reduced and a continuous moisturizing effect was not obtained. Since pickled reduced iron powder was used as the iron powder, the activity of the iron powder was high and the heat-generating reaction was violent and instantaneous.

As apparent from the foregoing illustration, according to the present invention, by using a metallic heat-generating material in which water is contained for a hair curler, the following effects can be attained.
(1) An easily portable hair curler is provided.
(2) Handling is simplified.
(3) The cost is reduced.
(4) The metallic heat-generating material capable of automatically generating heat can be thrown away after use.
(5) The hair is not damaged.
(6) The hair can be curled in a short time.
(7) The hair curler of the present invention has an excellent exchangeability not realized by the conventional portable metallic heat generation type hair curler.

## Claims

1. A heat-generating device (1) for a portable hair curler which comprises a heat-generating agent (3) containing water, characterised in that it further comprises iron powder, a water-retaining agent for retaining the water and a metal halide, the heat-generating agent being contained in an air-permeable bag (2) and the device being capable of generating a temperature of 70°C to 120°C and being capable of maintaining a temperature higher than 60°C for at least 20 minutes.

2. A heat-generating device for a portable hair curler according to claim 1, wherein the water-retaining agent is composed of a zeolite.

3. A heat-generating device for a portable hair curler according to claim 1, wherein the halide is sodium chloride (NaCl).

4. A heat-generating device for a portable hair curler according to claim 1, wherein the heat-generating agent further comprises an agent for controlling a generation of hydrogen.

5. A heat-generating device for a portable hair curler according to claim 4, wherein the agent for controlling the generation of hydrogen is sodium hydroxide (NaOH).

6. A heat-generating device for a portable hair curler according to claim 1, wherein an amount of water contained in the heat-generating agent is about 20 to about 50% by weight based on an amount of the iron powder contained.

7. A heat-generating device for a portable hair curler according to claim 1, wherein the heat-generating agent comprises 40 to 70% of iron powder, 15 to 25% of a zeolite, 2 to 5% of NaCl, 0.1 to 0.5% of NaOH, 1 to 3% of active carbon and 14 to 20% of water.

8. A heat-generating device for a portable hair curler according to claim 1, which is contained in an air-impermeable vessel (10).

9. A heat-generating device for a portable hair curler according to claim 1, which is contained in said portable hair curler.

10. A heat-generating device for a portable hair curler according to claim 1, which is contained in an air-impermeable vessel (10) together with said hair curler containing the heat-generating device therein.

11. A heat-generating device for a portable hair curler according to claim 9, wherein the hair curler comprises a hollow cylindrical hair-curling guide cylinder (4) having vent holes (7), a cap (13) dismountably fitted at an opening at an end of said cylinder and a cover (8) for holding wound hair on said cylinder.

12. A heat-generating device for a portable hair curler according to claim 11, wherein the hair curler further comprises comb tooth-like projections (6) formed on the outer surface of the hair-curling guide cylinder and projections formed on the inner surface of the cover and confronting said projections formed on the outer surface of the hair-curling guide cylinder.

13. A heat-generating device according to claim 1 wherein the halide is present in an amount of 2 - 5%.

## Patentansprüche

1. Wärmeerzeugende Vorrichtung (1) für einen tragbaren Lockenwickler mit einem wärmeerzeugenden Mittel (3), das Wasser enthält, dadurch gekennzeichnet, daß es weiterhin Eisenpulver, ein wasserhaltendes Mittel zum Halten des Wassers und ein Metallhalogenid umfaßt, wobei das wärmeerzeugende Mittel in einem luftdurchlässigen Beutel (2) enthalten ist und die Vorrichtung eine Temperatur von 70°C bis 120°C erzeugen kann und eine Temperatur von mehr als 60°C über wenigstens 20 Minuten aufrechterhalten kann.

2. Wärmeerzeugende Vorrichtung für einen tragbaren Lockenwickler nach Anspruch 1, bei der das wasserhaltende Mittel aus einem Zeolit besteht.

3. Wärmeerzeugende Vorrichtung für einen tragbaren Lockenwickler nach Anspruch 1, bei der das Halogenid Natriumchlorid (NaCl) ist.

4. Wärmeerzeugende Vorrichtung für einen tragbaren Lockenwickler nach Anspruch 1, bei der das wärmeerzeugende Mittel weiterhin ein Mittel zum Steuern der Erzeugung von Wasserstoff umfaßt.

5. Wärmeerzeugende Vorrichtung für einen tragbaren Lockenwickler nach Anspruch 4, bei der das Mittel zum Steuern der Erzeugung von Wasserstoff Natriumhydroxid (NaOH) ist.

6. Wärmeerzeugende Vorrichtung für einen tragbaren Lockenwickler nach Anspruch 1, bei der die Menge an Wasser, die im wärmeerzeugenden Mittel enthalten ist, etwa 20 bis etwa 50 Gew.-% auf der Grundlage der enthaltenen Eisenpulvermenge beträgt.

7. Wärmeerzeugende Vorrichtung für einen tragbaren Lockenwickler nach Anspruch 1, bei der das wärmeerzeugende Mittel 40 bis 70 % Eisenpulver, 15 bis 25 % Zeolit, 2 bis 5 % NaCl, 0,1 bis 0,5 % NaOH, 1 bis 3 % Aktivkohle und 14 bis 20 % Wasser umfaßt.

8. Wärmeerzeugende Vorrichtung für einen tragbaren Lockenwickler nach Anspruch 1, die in einem luftundurchlässigen Behälter (10) enthalten ist.

9. Wärmeerzeugende Vorrichtung für einen tragbaren Lockenwickler nach Anspruch 1, welche im tragbaren Lockenwickler enthalten ist.

10. Wärmeerzeugende Vorrichtung für einen tragbaren Lockenwickler nach Anspruch 1, welche in einem luftundurchlässigen Behälter (10) zusammen mit dem Lockenwickler enthalten ist, der die wärmeerzeugende Vorrichtung enthält.

11. Wärmeerzeugende Vorrichtung für einen tragbaren Lockenwickler nach Anspruch 9, bei der der Lockenwickler einen hohlzylindrischen Lockenwickelführungszylinder (4) mit Belüftungslöchern (7), eine Kappe (13), die abnehmbar auf eine Öffnung an einem Ende des Zylinders gepaßt ist, und eine Abdeckung (8) zum Halten des auf den Zylinder gewickelten Haars umfaßt.

12. Wärmeerzeugende Vorrichtung für einen tragbaren Lockenwickler nach Anspruch 11, bei der der Lockenwickler weiterhin kammzinkenartige Vorsprünge (6), die an der Außenfläche des Lockenwickelführungszylinders ausgebildet sind, und Vorsprünge umfaßt, die an der Innenfläche der Abdeckung ausgebildet sind und den Vorsprüngen gegenüberstehen, die an der Außenfläche des Lockenwickelführungszylinders ausgebildet sind.

13. Wärmeerzeugende Vorrichtung für einen tragbaren Lockenwickler nach Anspruch 1, bei der das Halogenid in einer menge von 2 bis 5 % vorliegt.

## Revendications

1. Dispositif (1) générateur de chaleur pour un bigoudi comprenant un agent (3) générateur de chaleur contenant de l'eau, caractérisé par le fait qu'il comprend en outre de la poudre de fer, un agent de rétention d'eau pour retenir l'eau et un halogénure métallique, l'agent générateur de chaleur étant contenu dans un sac (2) perméable à l'air et le dispositif étant capable de générer une température de 70°C à 120°C et de maintenir une température supérieure à 60°C pendant au moins 20 minutes.

2. Dispositif générateur de chaleur pour un bigoudi selon la revendication 1, dans lequel l'agent de rétention d'eau est constitué d'une zéolite.

3. Dispositif générateur de chaleur pour un bigoudi selon la revendication 1, dans lequel l'halogénure est du chlorure de sodium (NaCl).

4. Dispositif générateur de chaleur pour un bigoudi selon la revendication 1, dans lequel l'agent générateur de chaleur comprend en outre un agent pour contrôler la génération d'hydrogène.

5. Dispositif générateur de chaleur pour un bigoudi selon la revendication 4, dans lequel l'agent de contrôle de génération d'hydrogène est de l'hydroxyde de sodium (NaOH).

6. Dispositif générateur de chaleur pour un bigoudi selon la revendication 1, dans lequel la quantité d'eau contenue dans l'agent générateur de chaleur est d'environ 20 à 50% en poids par rapport à la quantité du contenu en poudre de fer.

7. Dispositif générateur de chaleur pour un bigoudi selon la revendication 1, dans lequel l'agent générateur de chaleur comprend de 40 à 70% de poudre de fer, de 15 à 25% d'une zéolite, de 2 à 5% de NaCl, de 0,1 à 0,5% de NaOH, de 1 à 3% de carbone actif et de 14 à 20% d'eau.

8. Dispositif générateur de chaleur pour un bigoudi selon la revendication 1, qui est contenu dans un réservoir (10) imperméable à l'air.

9. Dispositif générateur de chaleur pour un bigoudi selon la revendication 1, qui est contenu à l'intérieur dudit bigoudi.

10. Dispositif générateur de chaleur pour un bigoudi selon la revendication 1, qui est contenu dans un réservoir (10) imperméable à l'air avec ledit bigoudi contenant le dispositif générateur de chaleur.

11. Dispositif générateur de chaleur pour un bigoudi selon la revendication 9, dans lequel le bigoudi comporte un cylindre-guide (4) de mèche à friser dont l'intérieur creux est cylindrique ayant des trous d'évacuation d'air (7), un capuchon (13) démontable monté à une extrémité ouvrable dudit cylindre (4) et un chapeau (8) pour maintenir la mèche de cheveux enroulée sur ledit cylindre.

12. Dispositif générateur de chaleur pour un bigoudi selon la revendication 11, dans lequel ledit bigoudi comprend en outre des projections (6) en forme de dents de peigne formées sur la face extérieure du cylindre-guide de mèche de cheveux et des projections formées sur la face interne du couvercle et faisant face auxdites projections formées sur la face extérieure du cylindre-guide de mèche de cheveux.

13. Dispositif générateur selon la revendication 1, dans lequel l'halogénure est présent en quantité de 2 à 5%.
